# EUROPEAN PATENT SPECIFICATION

(11) **EP 1 812 070 B1**
(45) Date of publication and mention of the grant of the patent: **02.10.2013**
(21) Application number: 05807963.3
(22) Date of filing: 09.11.2005
(51) Int. Cl.: A61K 45/06, A61P 25/14, A61K 31/165, A61K 31/198

(54) **TREATMENT OF RESTLESS LEGS SYNDROME**
BEHANDLUNG DES SYNDROMS DER RUHELOSEN BEINE
TRAITEMENT DU SYNDROME DES JAMBES SANS REPOS

(30) Priority: 10.11.2004 US 626447 P
(43) Date of publication of application: 01.08.2007
(73) Proprietor: Orion Corporation, 02200 Espoo (FI)
(72) Inventor: ELLMEN, Juha, FI-20320 Turku (FI); KARVINEN, Johanna, FI-21260 Raisio (FI); VAHTERISTO, Mikko, FI-70500 Kuopio (FI)
(74) Representative: Sexton, Jane Helen
(86) International application number: PCT/FI2005/000478
(87) International publication number: WO 2006/051154

(56) References cited:
- EP-A- 1 588 704
- US-A1- 2004 166 159

## Description

### FIELD OF THE INVENTION

The present invention relates to the combination of a catechol-O-methyl transferase (COMT) inhibitor, a peripheral decarboxylase (DDC) inhibitor and a dopamine precursor, for use in a method of the treatment of restless legs syndrome (RLS).

### BACKGROUND OF THE INVENTION

RLS, also known as Ekbom's syndrome, is a neurological disorder and has an estimated prevalence of 2.5 to 15% in the adult population; the prevalence increases with age and is higher in women than in men. The condition is characterized by an irresistible urge to move the legs (akathisia), accompanied by other unpleasant sensations deep within the legs. The symptoms, which may extend to involve the arms or the trunk, are relieved by movement. The circadian rhythm seems to influence the subjective sensory symptoms of RLS and the associated periodic limb movements (PLM), which occur in approximately 80% of RLS patients. They occur primarily in the evening and at night, and the likelihood of symptoms increases with the relaxation of the patient.

The idiopathic or primary form of RLS occurs without any indication of other disease processes. Secondary or symptomatic RLS is known to occur in relation to a number of other medical conditions. The conditions that have been found to be clearly associated with symptomatic RLS include renal failure, as in end-stage renal disease, iron deficiency with or without anemia, pregnancy, and neuropathy or radiculopathy. Other associations of possible clinical importance include rheumatoid arthritis, diabetes, and both folate and magnesium deficiencies.

There is no officially established treatment of choice for RLS. For those who do receive RLS treatment, the medicinal products are usually prescribed "off-label". Dopaminergic products, especially dopamine agonists and dopamine precursors, including levodopa, appear to be the most common of the drug treatments currently used in RLA (Brodeur C, Montplaisir J, Godbout R, Marinier R, Neurology 1988:38:1845-1848, Kaplan PW, Allen RP, Buchholz DW, Walters JK, Sleep 1993; 16:717-723, Trenkwalder C, Stiasny K, Pollmächer T et al., Sleep 1995; 18(8): 681-688, Benes H, Kurella B, Kummer J, Kazenwadel J, Selzer R, Kohnen R, Sleep 1999; 22(8): 1073-1081). A limitation of levodopa/DDC inhibitor treatment in RLS is that it is relatively short-acting, due to the short half-life of levodopa in serum, and therefore it provides less symptomatic relief during the second half of the right (Benes H *et al., ibid*). Ashafq A, Sharif MD, Movement Disorders 2003; 17(2): 421 discloses the use of a COMT inhibitor in combination with levodopa in the treatment of secondary RLS after renal failure in a patient with polycystic kidney disease where alleviation of symptoms was reported up to 5 hours with a combination of a COMT inhibitor and up to 200 mg of sustained release levodopa/carbidopa.

### BRIEF DESCRIPTION OF THE DRAWINGS

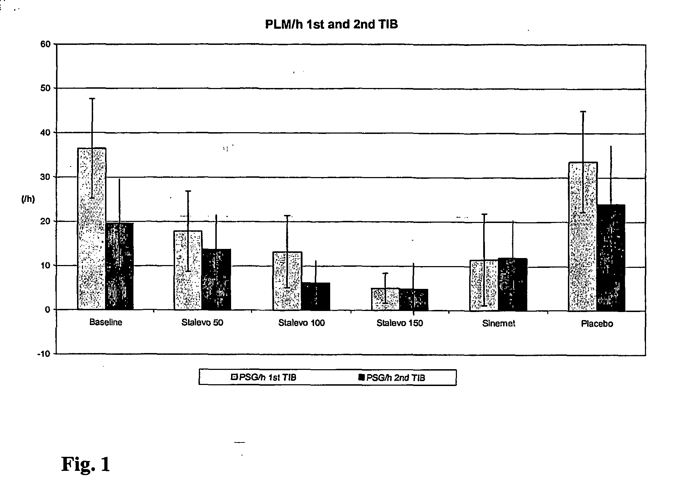

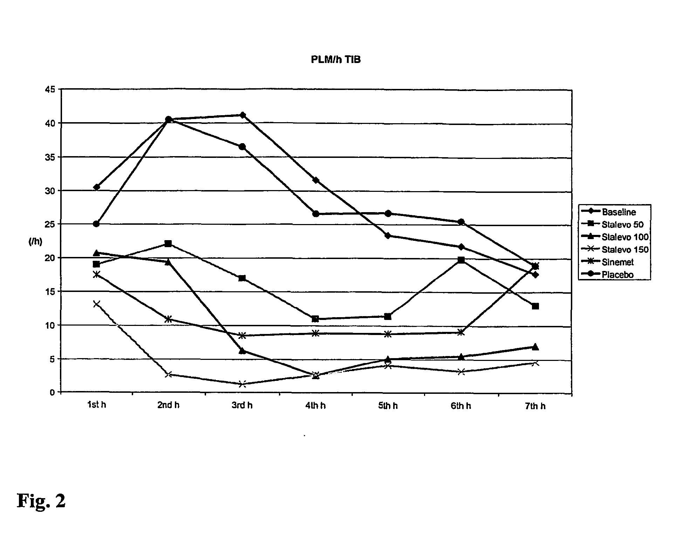

Figure 1 shows the PLM-indexes (periodic limb movement indexes) during the first (1^{st}) and second (2^{nd}) half of a polysomnography night in 28 patients with RLS undergoing a 7 period cross-over study. Figure 2 shows the hourly PLM-indexes during a polysomnography night in 28 patients with RLS undergoing a 5 period cross-over study. The abbreviations PSG and TIB mean polysomnography and total time in bed, respectively.

### DETAILS DESCRIPTION OF THE INVENTION

The applicants have discovered that when Stalevo®, a fixed combination of entacapone, a COMT inhibitor, levodopa and carbidopa, a DDC inhibitor, is given to patients with idiopathic RLS, the PLM index of said patients is reduced significantly also during the second half of the night. This is surprising, especially in view of the fact that the kinetics of immediate release levodopa alone or in combination with entacapone does not suggest such a long term action for levodopa therapy especially with such low doses. The effect of Stalevo® containing 100 and 150 mg of levodopa lasted up to 6 and 7 hours, respectively, and both were more effectively than standard levodopa/DDCI (Levodopa/DDC inhibitor) during the 4 last hours of the night (Figure 2). The effect of Stalevo® containing 50 mg of levodopa was similar to standard levodopa/DDCI containing 100 mg of levodopa during this period (Figure 2). The applicants were also surprised to observe that a single dose of Stalevo® containing 150 mg of levodopa was able to control, better than placebo, subjective symptoms of RLS for the week between the doses (Table 1).

Thus, COMT inhibitors, such as entacapone, and their pharmacologically acceptable esters or salts, can be used for the treatment of restless legs syndrome in combination with a dopamine precursor and a DDC inhibitor.

COMT inhibitors of the invention include entacapone and pharmaceutically acceptable esters or salts. Entacapone ((E)-N,N-diethyl-2-cyano-3-(3,4-dihydroxy-5-nitrophenyl)acrylamide) is described in US 5,446,194 and US 5,135,950, which are incorporated herein by reference. Stalevo®, the fixed combination of entacapone, levodopa and carbidopa, is disclosed in EP 1189608 B and Comtess®, the stand alone formulation of entacapone, is disclosed in EP 1112065 B. A dosage form comprising levodopa and carbidopa and entacapone and consisting of an immediate release and a controlled release component is disclosed in US 2004/0166459 A1. Tolcapone (3,4-dihydroxy-4'-methyl-5-nitrobenzophenone) is described in EP 237929 B.

DDC inhibitors include carbidopa. Levodopa and carbidopa are commercially available both as immediate release and slow release (depot) combination tablets sold in Europe under, for instance, the following trademarksL Nacom®, Sinemet® and Sinemet® Plus. Levodopa and benserazide are commercially available both as immediate release and slow release (depot) combination tablets, for instance, under the trademark Madopar®.

Dopamine precursors include, without limitation levodopa and its prodrugs, such as melevodopa, the methyl ester of levodopa, which is disclosed in EP 252290 B.

The present invention provides entacapone, carbidopa and levodopa for use in a method for the treatment of restless legs syndrome in a mammal comprising administering to the mammal in one or two daily doses an effective amount of entacapone, carbidopa and levodopa wherein the daily dose of levodopa is from 50mg to 600mg,
wherein the three active ingredients are combined in a single dosage form, and wherein
(i) the dose of entacapone is 200mg, the dose of carbidopa is 25mg, and the dose of levodopa is 100mg; or
(ii) the dose of entacapone is 200mg, the dose of carbidopa is 37.5mg, and the dose of levodopa is 150mg; or
(iii) the dose of levodopa is 100mg; or
(iv) the dose of levodopa is 150mg.

The precise amount of the drugs to be administered to a patient according to the present invention is dependent on numerous factors known to one skilled in the art, such as the compounds to be administered, the general condition of the patient, the severity of the condition to be treated and the desired duration of use. For example, Stalevo® and for combinations of entacapone (Comtess®/Comtan®) and levodopa/carbidopa, the usual daily dosage of levodopa will be from 50 mg to 300 mg but can be from 200 mg to 600 mg, divided into 1 to 4, preferably into 1 to 2 individual doses. However, based on the results obtained with the International Restless Legs Study Group (IRLSSG) rating scale, where single dose of Stalevo® containing 150 mg of levodopa was able to control the symptoms of RLS for a week and statistically significantly better than placebo, it is also possible to administer the drugs on-demand, i.e. when needed or required by the emerged symptoms, or once-a-week.

The catechol-O-methyl transferase inhibitor, peripheral decarboxylase inhibitor and dopamine precursor are combined in a single dosage form.

A kit is described containing a catechol-O-methyl transferase inhibitor, peripheral decarboxylase inhibitor and dopamine precursor. The kit may contain separate compartments to accommodate separate dosage forms. A patient may access the kit, for example, to take periodic doses of the active agents for the treatment of restless legs syndrom.

It is also possible to use a COMT inhibitor together with an immediate release or slow release combination of levodopa and carbidopa. In one embodiment of the invention, the daily dose of levodopa is divided into two individual doses, whereby the first does is an immediate release dosage form.

In another embodiment of the invention, the daily dose of levodopa is divided into two individual doses, whereby the second dose is a slow release dosage form.

In one embodiment of the invention, the drugs are for use by administration in a single dosage form comprising an immediate release and a slow release component. For example, both components may comprise the peripheral decarboxylase inhibitor and dopamine precursor, whereas the catechol-O-methyl transferase inhibitor may be contained only within one of the components or within both the immediate release and the slow release components.

In another embodiment the drugs are for use by administration on demand. The RLS symptoms may emerge unexpectedly once or twice a week for example when already in bed, during a flight, in a theatre or in a meeting. Dopamine precursor/peripheral decarboxylase inhibitor given in combination with entacapone has fast onset and long duration of action providing symptom relief within the first hour and lasting until morning or until the end of the flight, meeting or theatre.

In one embodiment of the invention, the peripheral decarboxylase inhibitor and dopamine precursor are for use by administration in a ratio from 1:1 to 1:40, such as from 1:4 to 1:10. For example, carbidopa and levodopa may be administered in a ratio of from 1:4 to 1:10, for instance in a ratio from 1:2 to 1:1.

For the purposes of this invention the term "treatment" means treatment in order to remedy or alleviate the symptoms of the disorder or the condition, and treatment in order to prevent the development or the exacerbation of the symptoms.

For the purposes of this invention the term "on-demand" means treatment taken when the RLS symptoms have unexpectedly emerged and disturb the ongoing activity such as trying to fall asleep, participating a meeting, travelling in an airplane or sitting in a theatre.

The term "immediate release component" used herein encompasses a component in a pharmaceutical formulation that would allow esentially similar release of levodopa from said formulation as immediate release Sinemet®.

The term "slow realease component" used herein encompasses a component in a pharmaceutical formulation that would allow essentially similar release of levodopa from said formulation as the slow release formulation Sinemet® Depot.

The invention will be further clarified by the following examples, which are intended to be purely exemplary of the invention.

### EXAMPLE 1

In this study the three strengths of the triple combination Stalevo® (i.e. levodopa:carbidopa:entacapone is 50 mg:12.5 mg:200 mg and 100 mg:25 mg:200 mg and 150 mg:37.5 mg:200 mg) were compared against placebo. Standard immediate release Sinemet® 100 (levodopa 100 mg + carbidopa 25 mg) was used as a positive control. At each treatment session, RLS patients received a single dose of treatment and were then monitored overnight for periodic limb movements by polysomnography (PSG). In clinical trials PSG has been a standard methodology for "objective" measurement of PLM in RLS patients.

The primary variable was periodic limb movements/h/total sleep time (PLM Index). The PLM Index has been one of the most common primary variables in short-term studies to document acute drug administration effects. Therefore it is regarded to be a validated, reliable and highly indicative variable to study the effects of Stalevo® in the study. To study the duration of the drug effect, the PLM Index was studied during the first and the second half of the total time in bed (TIB). Comparisons amongst all treatments were performed.

### Study outline

| | |
|---|---|
| Design: | A randomized, double-blind, placebo-controlled, cross-over, single-dose, dose-finding and proof-of-concept study with STALEVO® in RLS |
| Inclusion criteria: | Patients with idiopathic RLS according to IRLSSG criteria, who also had at least moderate PLMs (PLM/h in TST or TIB > 5/h) and were without current dopaminergic treatment for RLS |
| Methods: | Seven sleep laboratory nights with polysomnography (PSG) measurement for each subject including two baseline nights without any treatment and five nights with randomized study treatments (placebo, Stalevo® 50, Stalevo® 100, Stalevo® 150 and Sinemet®). A 4-8 day wash-out between the nights. The study treatment was given 30 min before bedtime. |
| Efficacy Variables: | I° PLM index (PLMs/h of total sleep time) |
| | II° PLM/h time in bed total and during the first and second half of the night. Other PSG variables, safety, subjective sleep quality |
| Sample size: | 28 |

| | |
|---|---|
| TST = total sleep time | |

### RESULTS

All three doses of Stalevo® were superior to placebo for all PLM variables studied. There was also a significant dose response between the three Stalevo® strengths. It is particularly noteworthy that Stalevo® 100 showed a prolonged activity on PLM in the latter part of the night, compared with standard levodopa 100 mg + DDC inhibitor (Figure 1).

### EXAMPLE 2

There was a difference in terms of the findings of the IRLSSG rating scale. Stalevo® containing 150 mg of levodopa was more effective than placebo, mean scores 9.3 (Stalevo® 150) and 10.9 (placebo). This scale is generally used in long-term studies as it measures overall RLS symptoms during the previous week. In the present study, the scale was used to assess RLS symptoms between the treatment periods. Summary information on the results from the IRLSSG rating scale is provided in Table 1.

**Table 1 IRLSSG Rating Scale**

| Descriptive statistics | | | | | | | |
|---|---|---|---|---|---|---|---|
| Variable | | Baseline | Stalevo®50 | Stalevo®100 | Stalevo®150 | Sinemet® | Placebo |
| Statistics | | | | | | | |
| Total score | N | 28 | 28 | 28 | 27 | 28 | 28 |
| | Mean | 11 | 10.4 | 11 | 9.3 | 10.3 | 10.9 |
| | Std Dev | 3.2 | 2.8 | 2.7 | 3.3 | 3.8 | 3.3 |
| | Minimum | 6 | 3 | 7 | 1 | 2 | 7 |
| | Median | 11.5 | 11 | 11 | 10 | 10.5 | 10 |
| | Maximum | 18 | 16 | 16 | 14 | 18 | 20 |

| Estimates | | | | | | | |
|---|---|---|---|---|---|---|---|
| Label | | Estimate | Std Err | t Value | Pr > \|t\| | Lower | Upper |
| Stalevo® 50 vs. Stalevo® | 100 | -0.6152 | 0.5733 | -1.07 | 0.2857 | -1.7522 | 0.5217 |
| Stalevo® 50 vs. Stalevo® | 150 | 1.0684 | 0.5796 | 1.84 | 0.0682 | -0.08120 | 2.2180 |
| Stalevo® 50 vs. Sinemet® | | 0.07036 | 0.5740 | 0.12 | 0.9027 | -1.0681 | 1.2089 |
| Stalevo® 50 vs. Placebo | | -0.4587 | 0.5756 | -0.80 | 0.4273 | -1.6002 | 0.6828 |
| Stalevo® 100 vs. Stalevo® | 150 | 1.6836 | 0.5793 | 2.91 | 0.0045 | 0.5346 | 2.8326 |
| Stalevo® 100 vs. Sinemet® | | 0.6856 | 0.5737 | 1.20 | 0.2348 | -0.4521 | 1.8233 |
| Stalevo® 100 vs. Placebo | | 0.1566 | 0.5748 | 0.27 | 0.7859 | -0.9834 | 1.2965 |
| Stalevo® 150 vs. Sinemet® | | -0.9980 | 0.5798 | -1.72 | 0.0882 | -2.1479 | 0.1519 |
| Stalevo® 150 vs. Placebo | | -1.5271 | 0.5814 | -2.63 | 0.0099 | -2.6801 | -0.3740 |
| Sinemet® vs. Placebo | | -0.5290 | 0.5740 | -0.92 | 0.3589 | -1.6675 | 0.6094 |

## Claims

1. Entacapone, carbidopa and levodopa for use in a method for the treatment of restless legs syndrome in a mammal comprising administering to the mammal in one or two daily doses an effective amount of entacapone, carbidopa and levodopa wherein the daily dose of levodopa is from 50mg to 600mg,
wherein the three active ingredients are combined in a single dosage form, and wherein
(i) the dose of entacapone is 200mg, the dose of carbidopa is 25mg, and the dose of levodopa is 100mg; or
(ii) the dose of entacapone is 200mg, the dose of carbidopa is 37.5mg, and the dose of levodopa is 150mg; or
(iii) the dose of levodopa is 100mg; or
(iv) the dose of levodopa is 150mg.

2. Entacapone, carbidopa and levodopa for use according to claim 1, wherein the dose of entacapone is 200mg, the dose of carbidopa is 25mg, and the dose of levodopa is 100mg.

3. Entacapone, carbidopa and levodopa for use according to claim 1, wherein the dose of entacapone is 200mg, the dose of carbidopa is 37.5mg, and the dose of levodopa is 150mg.

4. Entacapone, carbidopa and levodopa for use according to claim 1, wherein the dose of levodopa is 100mg.

5. Entacapone, carbidopa and levodopa for use according to claim 1, wherein the dose of levodopa is 150mg.

6. Entacapone, carbidopa and levodopa for use according to claim 1, wherein the restless legs syndrome is idiopathic.

7. Entacapone, carbidopa and levodopa for use according to any of claims 1 to 6, wherein carbidopa and levodopa may be administered in a ratio from 1:4 to 1:10.

8. Entacapone, carbidopa and levodopa for use according to any of claims 1 to 7, wherein the daily dose of levodopa is divided into two individual doses. wherein the first dose of levodopa is an immediate release dosage form.

9. Entacapone, carbidopa and levodopa for use according to any of claims 1 to 7, wherein the daily dose of levodopa is divided into two individual doses, wherein the second dose of levodopa is a slow release dosage form.

10. Entacapone, carbidopa and levodopa for use according to claim 1, wherein the active ingredients are for use in a method of administration once-a-week.

11. Entacapone, carbidopa and levodopa for use according to claim 1, wherein the active ingredients are for use in a method of administration on demand.

## Patentansprüche

1. Entacapon, Carbidopa und Levodopa zur Verwendung bei einem Verfahren zur Behandlung von Restless-Legs-Syndrom bei einem Säuger, umfassend dem Säuger Verabreichen, in ein oder zwei täglichen Dosen, einer wirksamen Menge von Entacapon, Carbidopa und Levodopa, wobei die tägliche Dosis von Levodopa 50 mg bis 600 mg beträgt,
wobei die drei Wirkstoffe in einer einzigen Darreichungsform kombiniert sind, und wobei
(i) die Entacapon-Dosis 200 mg beträgt, die Carbidopa-Dosis 25 mg beträgt und die Levodopa-Dosis 100 mg beträgt; oder
(ii) die Entacapon-Dosis 200 mg beträgt, die Carbidopa-Dosis 37,5 mg beträgt und die Levodopa-Dosis 150 mg beträgt; oder
(iii) die Levodopa-Dosis 100 mg beträgt; oder
(iv) die Levodopa-Dosis 150 mg beträgt.

2. Entacapon, Carbidopa und Levodopa zur Verwendung nach Anspruch 1, wobei die Entacapon-Dosis 200 mg beträgt, die Carbidopa-Dosis 25 mg beträgt und die Levodopa-Dosis 100 mg beträgt.

3. Entacapon, Carbidopa und Levodopa zur Verwendung nach Anspruch 1, wobei die Entacapon-Dosis 200 mg beträgt, die Carbidopa-Dosis 37,5 mg beträgt und die Levodopa-Dosis 150 mg beträgt.

4. Entacapon, Carbidopa und Levodopa zur Verwendung nach Anspruch 1, wobei die Levodopa-Dosis 100 mg beträgt.

5. Entacapon, Carbidopa und Levodopa zur Verwendung nach Anspruch 1, wobei die Levodopa-Dosis 150 mg beträgt.

6. Entacapon, Carbidopa und Levodopa zur Verwendung nach Anspruch 1, wobei das Restless-Legs-Syndrom idiopathisch ist.

7. Entacapon, Carbidopa und Levodopa zur Verwendung nach einem der Ansprüche 1 bis 6, wobei Carbidopa und Levodopa in einem Verhältnis von 1:4 bis 1:10 verabreicht werden können.

8. Entacapon, Carbidopa und Levodopa zur Verwendung nach einem der Ansprüche 1 bis 7, wobei die tägliche Dosis von Levodopa in zwei einzelne Dosen aufgeteilt ist, wobei die erste Levodopa-Dosis eine sofort freisetzende Darreichungsform ist.

9. Entacapon, Carbidopa und Levodopa zur Verwendung nach einem der Ansprüche 1 bis 7, wobei die tägliche Dosis von Levodopa in zwei einzelne Dosen aufgeteilt ist, wobei die zweite Levodopa-Dosis eine langsam freisetzende Darreichungsform ist.

10. Entacapon, Carbidopa und Levodopa zur Verwendung nach Anspruch 1, wobei die Wirkstoffe zur Verwendung bei einem Verfahren zur einmal wöchentlichen Verabreichung sind.

11. Entacapon, Carbidopa und Levodopa zur Verwendung nach Anspruch 1, wobei die Wirkstoffe zur Verwendung bei einem Verfahren zur Verabreichung nach Bedarf sind.

## Revendications

1. Entacapone, carbidopa et lévodopa pour une utilisation dans un procédé de traitement du syndrome des jambes sans repos chez un mammifère comprenant l'administration au mammifère en une ou deux doses quotidiennes d'une quantité efficace d'entacapone, de carbidopa et de lévodopa, dans lequel la dose quotidienne de lévodopa est de 50 mg à 600 mg,
dans lequel les trois principes actifs sont combinés en une forme galénique unique, et dans lequel
(i) la dose d'entacapone est de 200 mg, la dose de carbidopa est de 25 mg, et la dose de lévodopa est de 100 mg ; ou
(ii) la dose d'entacapone est de 200 mg, la dose de carbidopa est de 37,5 mg, et la dose de lévodopa est de 150 mg ; ou
(iii) la dose de lévodopa est de 100 mg ; ou
(iv) la dose de lévodopa est de 150 mg.

2. Entacapone, carbidopa et lévodopa pour une utilisation selon la revendication 1, dans laquelle la dose d'entacapone est de 200 mg, la dose de carbidopa est de 25 mg, et la dose de lévodopa est de 100 mg.

3. Entacapone, carbidopa et lévodopa pour une utilisation selon la revendication 1, dans laquelle la dose d'entacapone est de 200 mg, la dose de carbidopa est de 37,5 mg, et la dose de lévodopa est de 150 mg.

4. Entacapone, carbidopa et lévodopa pour une utilisation selon la revendication 1, dans laquelle la dose de lévodopa est de 100 mg.

5. Entacapone, carbidopa et lévodopa pour une utilisation selon la revendication 1, dans laquelle la dose de lévodopa est de 150 mg.

6. Entacapone, carbidopa et lévodopa pour une utilisation selon la revendication 1, dans laquelle le syndrome des jambes sans repos est idiopathique.

7. Entacapone, carbidopa et lévodopa pour une utilisation selon l'une quelconque des revendications 1 à 6, dans laquelle la carbidopa et la lévodopa peuvent être administrées en un rapport de 1/4 à 1/10.

8. Entacapone, carbodipa et lévodopa pour une utilisation selon l'une quelconque des revendications 1 à 7, dans laquelle la dose quotidienne de lévodopa est divisée en deux doses individuelles, dans laquelle la première dose de lévodopa est une forme galénique à libération immédiate.

9. Entacapone, carbodipa et lévodopa pour une utilisation selon l'une quelconque des revendications 1 à 7, dans laquelle la dose quotidienne de lévodopa est divisée en deux doses individuelles, dans laquelle la seconde dose de lévodopa est une forme galénique à libération prolongée.

10. Entacapone, carbidopa et lévodopa pour une utilisation selon la revendication 1, dans laquelle les principes actifs sont destinés à être utilisés dans un procédé d'administration une fois par semaine.

11. Entacapone, carbidopa et lévodopa pour une utilisation selon la revendication 1, dans laquelle les principes actifs sont destinés à être utilisés dans un procédé d'administration à la demande.
